# EUROPEAN PATENT APPLICATION

(11) **EP 2 759 834 A2**
(43) Date of publication of application: **30.07.2014**
(21) Application number: 12833567.6
(22) Date of filing: 23.08.2012
(51) Int. Cl.: G01N 33/53, G01N 27/30, G01N 33/48

(54) **BIOSENSOR AND MEASUREMENT APPARATUS FOR SAME**

(30) Priority: 23.09.2011 KR 20110096046
(71) Applicant: Ceragem Medisys Inc., Cheonan-si, Chungcheongnam-do 331-833 (KR)
(72) Inventor: LEE, Jin Woo, Cheonan-si Chungcheongnam-do 331-833 (KR); CHOI, Jae Kyu, Cheonan-si Chungcheongnam-do 331-833 (KR)
(74) Representative: Bridge, Kerry Ann
(86) International application number: PCT/KR2012/006725
(87) International publication number: WO 2013/042877

(57) **Abstract**

Provided is a biosensor and a measuring device which may be coupled with each other only when the biosensor and the measuring device mach with each other, thereby obtaining reliable measured results.

The measuring device having a receiving part in which the biosensor is inserted, includes exclusive coupling parts formed correspondingly at each contact surface at which the biosensor and the receiving part of the measuring device are in contact with each other, wherein the biosensor is inserted into the receiving part only when the exclusive coupling parts mate with each other.

## Description

### [Technical Field]

The present invention relates to a biosensor and a measuring device thereof, and more particularly, to a biosensor and a measuring device thereof which may be coupled with each other only when the biosensor and the measuring device thereof mach with each other, thereby providing improved usability and diagnostic performance.

### [Background Art]

A biosensor is a means which inspects properties or the like of a substance using a function of a living body. Since the biosensor uses a biological material, such as blood sugar and ketone, as a detecting device, it has excellent sensitivity and reaction specificity. Thus, the biosensor is widely used in the fields of medicine and pharmacy, such as clinical chemistry analysis, process instrumentation in bioindustry, environment measurement, and safety evaluation of a chemical substance, and the range of use thereof is being increased continuously. Particularly, the biosensor is frequently used in various self-tests, such as blood sugar measurement, pregnancy diagnosis, and urine examination, and fast disease diagnosis.

The biosensor may be classified into a biosensor used in an enzymatic analysis and a biosensor used in an immunoassay according to an analysis method, and also may be classified into an optical biosensor and an electrochemical biosensor according to a quantitative analysis method of a target substance in a biological sample.

The biosensor used in the enzymatic analysis uses a specific reaction between an enzyme and a substrate and between an enzyme and an inhibitor of enzyme reaction, and the biosensor used in the immunoassay uses a specific reaction between an antigen and an antibody. The optical biosensor is a method which measures a light transmittance, an optical density, or a change in wavelength and thus measures a concentration of a target material and which is most commonly used. The method using the optical biosensor has some advantages in that reaction mechanisms of various materials are already well known and it has a small deviation with respect to measurement time, because a measurement operation is performed after a reaction is achieved for a sufficient time. However, the optical biosensor has some disadvantages in that a longer measurement time and a larger amount of samples are required, compared the electrochemical biosensor, and also a measured result thereof is affected by turbidity of a sample, and it is difficult to miniaturize an optical part.

The electrochemical biosensor is a method which measures an electric signal obtained from a reaction and thus measures the concentration of the target material. The electrochemical biosensor may amplify a signal with only a thimbleful of the sample, may be easily miniaturized, may stably obtain a measurement signal, and also may be easily integrated with information communication equipment.

The electrochemical biosensor is mainly used in the blood sugar measurement. Here, an electric signal is generated by an electrochemical reaction occurring when a sample such as blood is introduced therein, and then transferred to a measuring device connected or coupled with the biosensor.

The biosensor generally has different properties according a production lot, and this may cause different result values with the same biological substance. In order to solve the problem, the measuring device coupled with the biosensor performs proper compensation according to the production lot in order to derive the same result values.

Most of such conventional biosensors generally have a thin stick shape. Particularly, since a portion thereof which is inserted into a slit of the measuring device is configured to have a similar structure, there is a problem that a biosensor having different properties may be inserted into the same measuring device, and thus a measurement error may occur.

As described above, since a compensation algorithm applied to the measuring device does not match with the inserted biosensor, the measuring device may output an erroneous measurement result, and thus an accident may occur at a medical site.

### [Disclosure]

### [Technical Problem]

The present invention is directed to providing a biosensor and a measuring device thereof which may be coupled with each other only when the biosensor and the measuring device thereof mach with each other, and thus may prevent generation of an erroneous measurement result.

### [Technical Solution]

One aspect of the present invention provides a measuring device having a receiving part in which a biosensor is inserted, including exclusive coupling parts formed correspondingly at each contact surface at which the biosensor and the receiving part of the measuring device are in contact with each other, wherein the biosensor is inserted into the receiving part only when the exclusive coupling parts mate with each other.

The exclusive coupling parts may include at least one protrusion configured to protrude toward the biosensor from an inner side surface of the receiving part which is the contact surface, and at least one coupling groove formed in an outer surface of the biosensor, which is the contact surface, to be concave in a shape corresponding to the protrusion, such that the protrusion is inserted, and to extend in a lengthwise direction in which the protrusion is inserted.

The exclusive coupling part may include at least one protrusion configured to protrude from an outer surface of the biosensor which is the contact surface; and at least one coupling groove formed in an inner side surface of the receiving part, which is the contact surface, to be concave in a shape corresponding to the protrusion, such that the protrusion is inserted, and to extend in a lengthwise direction in which the protrusion is inserted.

The protrusion may have a polygonal cross section.

The protrusion may have a streamlined cross section.

The biosensor may include a base substrate, a reaction substrate configured to be coupled to the base substrate, to generate a reaction signal according to a reaction with a target biological material, and to transfer the reaction signal to the electrically connected measuring device, an operation electrode and a reference electrode formed on the reaction substrate, and a capillary passage formed between the base substrate and the reaction substrate, and having a sample introduction portion opened at one side thereof and an air discharging portion provided at a middle portion thereof.

The measuring device may include an input part through which a user inputs information, a storing part configured to store data, a display part configured to display the data, and a control part configured to control each construction part according to the input from the user, data input and output of the storing part, and displaying of the display part according to a preset state.

Another aspect of the present invention provides a biosensor which is received in a measuring device having a receiving part, including a reaction substrate configured to generate a reaction signal according to a reaction with an introduced sample and transfer the reaction signal to the electrically connected measuring device, and a base substrate configured to be coupled with the reaction substrate and form a capillary passage including a reaction chamber, wherein an exclusive coupling part mating with the receiving part in an aspect of shape is provided at one side of the base substrate received in the receiving part, and the biosensor is received in the receiving part only when the exclusive coupling part mates with the receiving part in an aspect of shape.

The exclusive coupling part may be formed as at least one of at least one protrusion and at least one coupling groove.

A part of an entire area of the biosensor, which is received in the measuring device, may be configured so that a cross section thereof in a direction in which the biosensor is inserted into the measuring device is the same as a shape of an inner side surface of the receiving part of the measuring device.

### [Advantageous Effects]

As described above, since the biosensor and the measuring device thereof according to the present invention are configured to be coupled with each other only when the biosensor and the measuring device thereof mach with each other, it is possible to fundamentally prevent the measurement error which may occur when a different kind of biosensor is inserted into the measuring device, and thus to improve reliability of the product.

### [Description of Drawings]

FIG. 1 is a perspective view of a biosensor and a measuring device thereof according to a first embodiment of the present invention.
FIG. 2 is a view schematically illustrating the measuring device illustrated in FIG. 1.
FIG 3 is an exploded perspective view of the measuring device illustrated in FIG. 1.
FIG. 4 is a cross-sectional view illustrating a state in which the biosensor is coupled to the measuring device illustrated in FIG. 1.
FIG. 5 is a cross-sectional view illustrating a state in which a biosensor is coupled to a measuring device according to a second embodiment of the present invention.
FIG. 6 is a cross-sectional view illustrating a state in which a biosensor is coupled to a measuring device according to a third embodiment of the present invention.
FIG. 7 is a cross-sectional view illustrating a state in which a biosensor is coupled to a measuring device according to a fourth embodiment of the present invention.

### [Modes of the Invention]

Hereinafter, exemplary embodiments of the present invention will be described in detail with reference to the accompanying drawings.

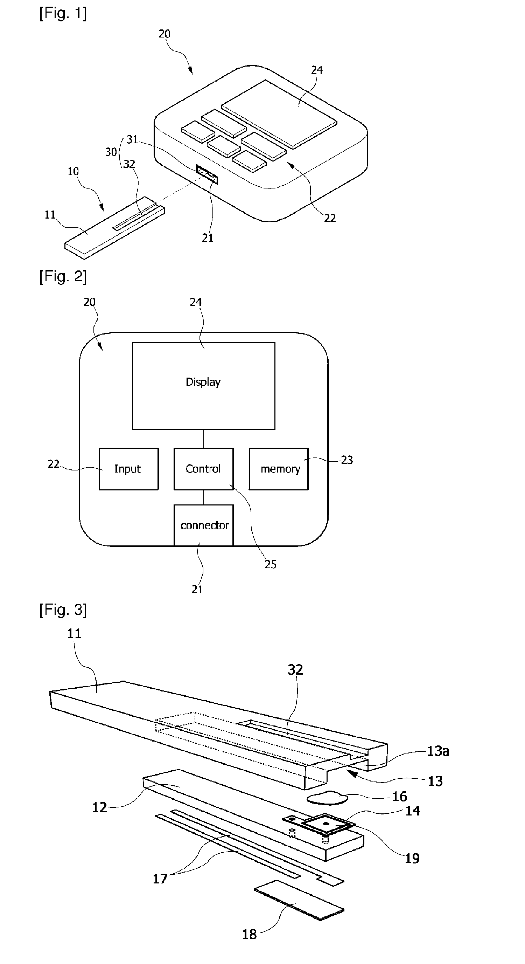

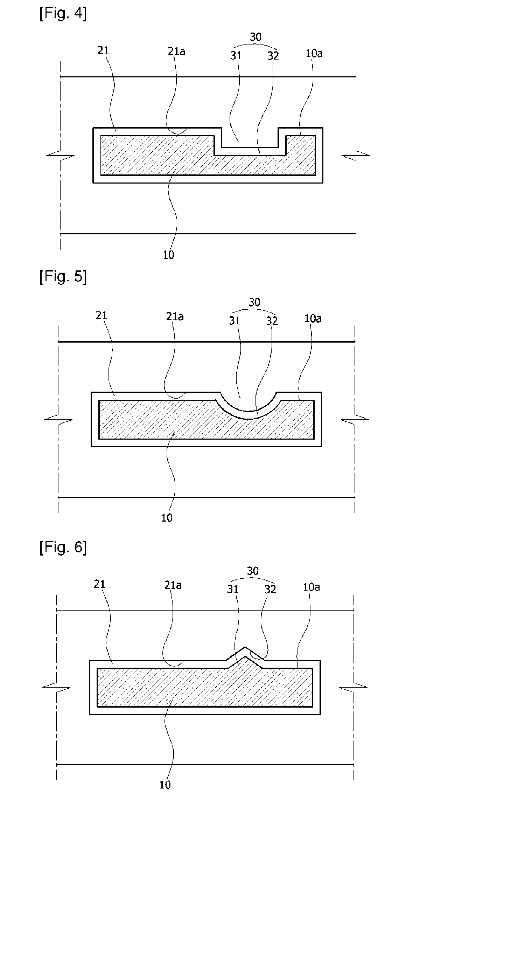

FIG. 1 is a perspective view of a biosensor and a measuring device thereof according to a first embodiment of the present invention, FIG. 2 is a view schematically illustrating the measuring device illustrated in FIG. 1, FIG 3 is an exploded perspective view of the measuring device illustrated in FIG. 1, and FIG. 4 is a cross-sectional view illustrating a state in which the biosensor is coupled to the measuring device illustrated in FIG. 1.

In one embodiment of the present invention, as illustrated in FIGS. 1 to 4, a biosensor and a measuring device thereof may include an exclusive coupling part 32 having a specific shape. The measuring device 20 may include a receiving part 21 in which the biosensor 10 is received. The receiving part 21 may include an exclusive coupling part 31 which matches with the exclusive coupling part 32 in an aspect of shape.

The biosensor 10 includes a base substrate 11 which forms a body, and a reaction substrate 12 which is coupled with the base substrate 11, generates a reaction signal corresponding to a reaction with an introduced sample, and then transmits the reaction signal to the electrically connected measuring device 20.

When the reaction substrate 12 is assembled to the base substrate 11, a capillary passage 13 is formed between the reaction substrate 12 and the base substrate 11. An opening portion may be formed at one side of the capillary passage 13 and constituted as a sample introduction portion 13a through which a sample is introduced. If the sample is introduced through the sample introduction portion 13a, the sample is transported through the capillary passage 13 by a capillary phenomenon. An air discharging portion (not shown) in communication with an outside may be formed at an area of the capillary passage 13. The air discharging portion serves to discharge air when the sample is sucked and thus enable fast sample suction. The air discharging portion may be formed at an area opposite to the sample introduction portion 13a or may be formed to be spaced apart in a predetermined distance from the sample introduction portion 13a.

The capillary passage 13 is formed at a gap between the base substrate 11 and the reaction substrate 12, which is defined when the reaction substrate 12 is assembled to the base substrate 11. In this space, an area in which a reaction electrode is formed on the reaction substrate 12 forms a reaction chamber. The reaction electrode may include an operation electrode 19 and a reference electrode 14, and may be immobilized so that a reagent 16 is coated thereon. An electrochemical reaction between the sample and the reagent 16 occurs at the reaction chamber, and thus a reaction signal is generated. The operation electrode 19 and the reference electrode 14 transfer the reaction signal to the measuring device 20 through signal transferring parts 17 which are electrically connected thereto. The signal transferring parts 17 formed of wires may be formed at the same surface as or an opposite surface to the operation electrode 19 and the reference electrode 14. In a case in which the signal transferring parts 17 are formed at the opposite surface to the operation electrode 19 and the reference electrode 14, the signal transferring parts 17 are electrically connected with the operation electrode 19 and the reference electrode 14 through a via hole. The reaction substrate 12 may be embodied as a printed circuit board (PCB) or a flexible PCB (FPCB), and the operation electrode 19, the reference electrode 14, and the signal transferring parts 17 may be plated thereon.

The receiving part 21 in which the biosensor 10 is received is formed at the measuring device 20. The measuring device 20 includes an input part 22 through which a user inputs information, a storing part 23 which stores data, a display part 24 which displays the data, and a control part 25 which controls each construction part according to the input from the user, data input and output of the storing part 23, displaying of the display part 24 according to a preset state, and so on.

The exclusive coupling parts 30 having shapes corresponding to each other are formed at a contact surface at which the biosensor 10 and the receiving part 21 of the measuring device 20 are in contact with each other, such that the biosensor 10 may be received (or inserted) into the receiving part 21 only when the exclusive coupling parts 30 mate and coincide with each other. That is, in a part of an entire area of the biosensor 10 which is received in the measuring device 20, the biosensor 10 may be received in the receiving part 21 of the measuring device 20 only when a cross section thereof in an insertion direction coincides with a shape of an inner side surface of the receiving part 21 of the measuring device 20.

As an example, the exclusive coupling parts 30 may be configured with at least one protrusion 31 formed to protrude toward the biosensor 10 from an inner side surface 21a of the receiving part 21, which is one of the contact surfaces between the biosensor 10 and the receiving part 21 of the measuring device 20, and a coupling groove 32 formed in an outer surface 10a of the biosensor 10, which is the other contact surface, to be concave corresponding to the protrusion 31 and to extend in a lengthwise direction in which the protrusion 31 is inserted.

The protrusion 31 has a polygonal cross section, and the coupling groove 32 is concave to have a corresponding cross section. The polygonal cross section may be a quadrangular cross section, as illustrated in the drawings. However, the present invention is not limited thereto, and may have all kinds of user's preferred polygonal cross sections, such as a triangular cross section, a pentagonal cross section, and a hexagonal cross section. Further, positions and the number thereof may differ so that the biosensor 10 is inserted into the receiving part 21 of the measuring device 20 only when the shapes, the positions, and the number of the exclusive coupling parts 30 coincide with each other. Further, as illustrated in the drawings, the exclusive coupling parts 30 are formed in an upper surface of the contact surface, but may be formed in both side surfaces or a lower surface. In an aspect of formation of the variously shaped exclusive coupling parts 30, the base substrate 11 may be formed of a synthetic resin, such as plastic, which is easily molded. Similarly, at least a part of the receiving part 21 including the exclusive coupling part 30 may be formed of the synthetic resin. In this case, the exclusive coupling parts 30 may be integrally embodied at the receiving part 21.

As described above, according to the first embodiment of the present invention, 10 and the measuring device 20 are configured so that the biosensor 10 is received in the measuring device 20 only when biosensor 10 and the measuring device 20 have the exclusive coupling parts 30 matching with each other. When the shapes, the numbers, or the positions of the protrusion 31 and the coupling groove 32 do not coincide with each other, the biosensor 10 may not be received in the measuring device 20, and thus it is possible to fundamentally prevent generation of measurement errors. If the biosensor 10 is received in the receiving part 21 of the measuring device 20 and then the sample is introduced, the reagent and the sample react with each other on the operation electrode 19 and the reference electrode 14 to generate a reaction signal, and the reaction signal is transferred to the control part 25 via the signal transferring parts 17 and the receiving part 21 (a connecting means provided in the receiving part 21) of the measuring device 20. The control part 25 applies a compensation algorithm to measured results according to the reaction signal, and displays result values thereof numerically on the display part 24.

Like this, the biosensor 10 and the measuring device 20 are configured to have different exclusive coupling parts 30 from each other according to the kinds thereof, such that only the biosensor 10 and the measuring device 20 matching with each other may be coupled with each other, and the coupling between the different kinds of biosensor and measuring device from each other is fundamentally prevented. Therefore, it is possible to prevent the measurement error and thus always provide reliable measured results.

Similarly, in a biosensor and a measuring device thereof according to a second embodiment of the present invention, as illustrated in FIG. 5, a protrusion 31 constituting exclusive coupling parts 30 is formed to have a streamlined cross section, and a coupling groove 32 is formed to be concave in a streamlined shape corresponding to a shape of the protrusion 31 and to extend in a lengthwise direction in which the protrusion 31 is inserted.

Further, positions and the number thereof may differ so that the biosensor 10 is received (or inserted) into the receiving part 21 of the measuring device 20 only when the shapes, the positions, and the number of the exclusive coupling parts 30 coincide with each other. Further, the exclusive coupling parts 30 of the present invention are formed in an upper surface of the contact surface, but may formed in both side surfaces or a lower surface.

The structure and operation of the exclusive coupling parts 30 are the same as in the first embodiment, except that the exclusive coupling part 30 has the streamlined cross section.

In a biosensor and a measuring device thereof according to a third embodiment of the present invention, as illustrated in FIG. 6, exclusive coupling parts 30 having shapes corresponding to each other are formed at each contact surface at which the biosensor 10 and a receiving part 21 of the measuring device 20 are in contact with each other, such that the biosensor 10 is received (or inserted) in the receiving part 21 only when the exclusive coupling parts 30 mate and coincide with each other.

The exclusive coupling parts 30 are configured with at least one protrusion 31 formed to protrude from an outer surface 10a of the biosensor 10, which is one of the contact surfaces between the biosensor 10 and the receiving part 21 of the measuring device 20, and a coupling groove 32 formed in an inner side surface 21a of the receiving part 21, which is the other contact surface, to be concave in a shape corresponding to the protrusion 31, such that the protrusion 31 may be inserted therein, and to extend in a lengthwise direction in which the protrusion 31 is inserted. In a case in which the exclusive coupling part 30 protrudes from the outer surface 10a of the biosensor 10, the measuring device 20 may recognize that the biosensor 10 is inserted into the measuring device 20, when the protruded protrusion 31 of the biosensor 10 is coupled into the coupling groove 32 of the measuring device 20, and then may start an operation thereof. To this end, a recognizing means for recognizing the protrusion 31 may be provided at at least a part of the coupling groove 32.

The protrusion 31 has a polygonal cross-sectional shape, and the coupling groove 32 is concave to have a corresponding shape. The polygonal cross section may be a triangular cross section, as illustrated in the drawings. However, the present invention is not limited thereto, and may have all kinds of user's preferred polygonal cross sections, such as a quadrangular cross section, a pentagonal cross section, and a hexagonal cross section. Further, positions and the number thereof may differ so that the biosensor 10 is inserted into the receiving part 21 of the measuring device 20 only when the shapes, the positions, and the number of the exclusive coupling parts 30 coincide with each other. Further, as illustrated in the drawings, the exclusive coupling parts 30 are formed in an upper surface of the contact surface, but may formed in both side surfaces or a lower surface.

The structure and operation of the exclusive coupling parts 30 are the same as in the first embodiment, except that the protrusion 31 is formed on the outer surface 10a of the biosensor 10, and the coupling groove 32 is in the inner side surface 21a of the receiving part 21.

Further, although not illustrated in the drawings, the shapes of the exclusive coupling parts 30 described in the first, second, and third embodiments of the present invention may be formed mixedly. That is, a mixed structure in which an exclusive coupling part 30 having a quadrangular cross section and another exclusive coupling part 30 having another polygonal cross section or the streamlined cross section are mixed may be provided. Alternatively, a structure in which the protrusion 31 formed on the inner side surface of the receiving part 21 (the first and second embodiments), and another structure in which the protrusion 31 is formed on the outer surface 10a of the biosensor 10 (the third embodiment) may be mixed.

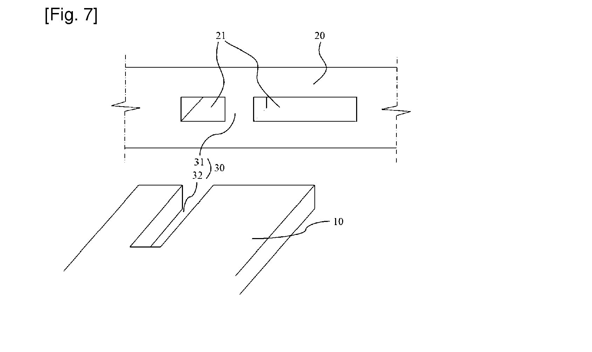

In a biosensor and a measuring device thereof according to a fourth embodiment of the present invention, as illustrated in FIG. 7, a protrusion 31 of an exclusive coupling part 30 may be formed in at least one pillar shape configured to obstruct a predetermined area of a receiving part 21, or another equivalent level of shapes. Therefore, a coupling groove 32 of the biosensor 10 matching with the measuring device 20 may be formed at an end area of the biosensor 12 to be concave by a predetermined depth according to the shape and the position of the protrusion 31.

Although a few embodiments of the present invention have been shown and described, it would be appreciated by those skilled in the art that changes may be made in these embodiments without departing from the principles and spirit of the invention, the scope of which is defined in the claims and their equivalents.

## Claims

1. A measuring device (20) having a receiving part (21) in which a biosensor (10) is inserted, comprising exclusive coupling parts (30) formed correspondingly at each contact surface at which the biosensor (10) and the receiving part (21) of the measuring device (20) are in contact with each other,
wherein the biosensor (10) is inserted into the receiving part (21) only when the exclusive coupling parts (30) mate with each other.

2. The measuring device of claim 1, wherein the exclusive coupling parts (30) comprise:
at least one protrusion (31) configured to protrude toward the biosensor from an inner side surface (21a) of the receiving part (21) which is the contact surface; and
at least one coupling groove (32) formed in an outer surface (10a) of the biosensor, which is the contact surface, to be concave in a shape corresponding to the protrusion (31), such that the protrusion (31) is inserted, and to extend in a lengthwise direction in which the protrusion (31) is inserted.

3. The measuring device of claim 1, wherein the exclusive coupling parts (30) comprise:
at least one protrusion (31) configured to protrude from an outer surface (10a) of the biosensor (10) which is the contact surface; and
at least one coupling groove (32) formed in an inner side surface (21a) of the receiving part (21), which is the contact surface, to be concave in a shape corresponding to the protrusion (31), such that the protrusion (31) is inserted, and to extend in a lengthwise direction in which the protrusion (31) is inserted.

4. The measuring device of claim 2 or 3, wherein the protrusion (31) has a polygonal cross section.

5. The measuring device of claim 2 or 3, wherein the protrusion (31) has a streamlined cross section.

6. The measuring device of any one of claims 1 to 3, wherein the biosensor (10) comprises:
a base substrate (11);
a reaction substrate (12) configured to be coupled to the base substrate (11), to generate a reaction signal according to a reaction with a target biological material, and to transfer the reaction signal to the electrically connected measuring device (20);
an operation electrode (19) and a reference electrode (14) formed on the reaction substrate; and
a capillary passage (13) formed between the base substrate (11) and the reaction substrate (12), and having a sample introduction portion (13a) opened at one side thereof and an air discharging portion provided at a middle portion thereof.

7. The measuring device of any one of claims 1 to 3, wherein the measuring device (20) comprises:
an input part (22) through which a user inputs information;
a storing part (23) configured to store data;
a display part (24) configured to display the data; and
a control part (25) configured to control each construction part according to the input from the user, data input and output of the storing part, and displaying of the display part according to a preset state.

8. A biosensor (10) which is received in a measuring device (20) having a receiving part (21), comprising:
a reaction substrate (12) configured to generate a reaction signal according to a reaction with an introduced sample and transfer the reaction signal to the electrically connected measuring device (20); and
a base substrate (11) configured to be coupled with the reaction substrate (12) and form a capillary passage (13) comprising a reaction chamber,
wherein an exclusive coupling part (30) mating with the receiving part (21) in an aspect of shape is provided at one side of the base substrate received in the receiving part (21), and the biosensor (10) is received in the receiving part (21) only when the exclusive coupling part (30) mates with the receiving part (21) in an aspect of shape.

9. The biosensor of claim 8, wherein the exclusive coupling part (30) is formed as at least one of at least one protrusion (31) and at least one coupling groove (32).

10. The biosensor of claim 8, wherein a part of an entire area of the biosensor (10), which is received in the measuring device (20), is configured so that a cross section thereof in a direction in which the biosensor (10) is inserted into the measuring device (20) is the same as a shape of an inner side surface of the receiving part (21) of the measuring device (20).
